# EUROPEAN PATENT APPLICATION

(11) **EP 1 355 146 A2**
(43) Date of publication of application: **22.10.2003**
(21) Application number: 03008552.6
(22) Date of filing: 14.04.2003
(51) Int. Cl.: G01N 33/00

(54) **Use of the multipin platform as anchor device**

(30) Priority: 15.04.2002 US 372527 P; 11.04.2003 US 411693
(71) Applicant: Becton, Dickinson and Company, Franklin Lakes, New Jersey 07417 (US)
(72) Inventor: Sanchez-Martinez, Demetrio, Lynnfield, Massachusetts (US)
(74) Representative: von Kreisler, Alek, Dipl.-Chem.

(57) **Abstract**

A device for performing diagnostic and analytical assays is provided which includes a platform (4) and a plurality of pins (6) extending therefrom, the pin s being adapted to be insertable into a reagent repository (8) and having a ligand (10) attached thereto, the ligand being reactive with a target reagent (12) to form a complex when placed in contact therewith, the ligand portion of the complex remaining substantially attached to the pins during an analytic measurement. The pins may further include sensors and/or transmitters for real-time monitoring and control of a reaction.

## Description

### BACKGROUND OF THE INVENTION

### 1. Field of the Invention

The present invention relates to a device including a plurality of pins adapted to be inserted into a reagent depository. More specifically, the invention relates to a multi-pin device for analytical measurements wherein the pins include a ligand attached thereto which is reactive with a reagent when placed in contact therewith and wherein the pins may include sensors and/or transmitters of a signal.

### 2. Description of Related Art

Present methods of drug discovery involve the screening of large numbers of compounds in order to identify those compounds having a desired biological activity. Assays used to assess the biological activity include receptor binding assays, enzyme assays or cell-cell interaction assays which are readily carried out in microtiter plates in reaction volumes of from 50 to 250 microliters and can easily be automated. Automation and miniaturization of these test systems allows the sample throughput to be high.

Several groups have undertaken efforts to increase the automation of analytical test systems in order to increase sample throughput. For example, automated systems have been developed to transfer fluid samples from repositories, such as test tubes, vials or wells, to receptacles or surfaces. To this end, it is known to use a multi-pin device for transferring small fluid volumes containing test compounds into the wells of a microtiter plate. The pins may be treated to produce hydrophilic tips and a hydrophobic shaft so that the fluid sample containing the test compound is physically constrained by surface tension to the hydrophilic tip. Transfer of the fluid from a pin of the device into the wells of the assay plate occurs by moving the pins into close proximity to the assay plate.

It is also known to use a reagent transfer device for transferring a plurality of reagent samples onto a deposit surface. The device includes a transfer member having a transfer surface with a two-dimensional array of pins extending therefrom. Reagents are deposited on the pins by adhesion to the outer surface of the pins and transferred to a deposit surface where they are deposited by adhesion to the deposit surface.

Each of the systems described above is suitable for transferring fluid samples into the wells of a multi-well plate, such as a microtiter assay plate. However, none of these systems provides for the use of the pins as a transmitter and/or sensor of a detectable signal generated in the wells of the multi-well assay plate. Moreover, none of the test systems discloses using the pins as a solid support on which to adsorb or chemically bind a ligand which participates in the generation of a detectable signal.

It has recently been shown that a fluorescently labeled substrate molecule may by physically attached to a plurality of plastic pins extending from a lid for a microtiter plate. Samples to be assayed for protease activity are placed in the wells of the plate. The substrate-coated pins are immersed into the solution in the wells. During an incubation period, any proteases present in the sample will cleave the labeled substrate, which causes the substrate to be released from the pin into the solution in the well. At the end of the incubation period, the pins are removed from the wells and the fluorescent substrate released from the pin is detected with a plate reader. A disadvantage of this method is that it is limited to the detection and quantitation of protease enzymes and their inhibitors. The pins are used as a solid phase to anchor a substrate in place and to remove the unreacted substrate to prevent it from interfering with the reaction signal. While the target enzyme reacts with the substrate on the pin, neither the target nor the cleaved substrate remain associated with the pin following the reaction. This limits the number of assay formats possible with this particular system. Furthermore, the system is limited in that the detectable product can only be measured in the solution in the wells and not on the pin itself. Lastly, the pins are not disclosed as including sensors and/or transmitters for direct real-time monitoring and control of a reaction.

It would be an advantage, therefore, to provide a test system wherein the pins were useful as a sensor of a detectable signal generated during an analytical method and where the pins could also transmit signals for altering reaction conditions in given wells of a multi-well plate. Such transmission may be of electricity or heat, for example. Moreover, it would be advantageous to provide a test system where the pins serve as a solid support for performing a reaction thereon.

### 3. Summary of the Invention

The present invention provides a platform assembly for conducting analytical measurements including a platform and a plurality of pins extending from the platform, the pins being adapted to be insertable into a reagent repository and including a ligand attached thereto, the ligand being reactive with a target reagent to form a complex when placed in contact therewith, the ligand portion of the complex remaining substantially attached to the pins during an analytic measurement. The pins of the platform assembly are particularly useful for performing a reaction thereon. The pins may include sensors and/or transmitters of a signal which provide for real-time monitoring and control of a reaction. For example, a ligand may be present on the pin in an adsorbed or chemically bound form for subsequent reaction with reagents present in the wells of the multi-well assay plate. As such, a complex formed on a given pin which releases a detectable signal may be measured directly by using the pin as a sensor.

A further aspect of the present invention relates to an analytical measurement method which includes the steps of providing a platform assembly including a platform and a plurality of pins which are adapted to be insertable into a reagent repository; attaching a ligand to the pins for conducting analytical measurements; contacting the attached ligand with a target reagent in a reagent repository to form a complex, the ligand portion of the complex remaining substantially attached to the pins during an analytic measurement; and measuring a signal which correlates with the analytical measurement. It is noted that, in addition to the target reagent, the pins may be brought into contact with a further series of reagents, as desired, to facilitate the generation of a signal. Moreover, the method may further include the steps of washing or incubating as desired.

Further encompassed by the present invention is a method of screening to identify a substance capable of binding to or modulating the activity of a ligand including the steps of providing a platform assembly including a platform and a plurality of pins which are adapted to be insertable into a reagent repository; attaching a ligand to the pins; exposing the attached ligand to a substance suspected of being capable of binding to or modulating the activity of the ligand to form a complex, the ligand portion of the complex remaining substantially attached to the pins during the screening process; and determining whether the substance binds to or modulates the activity of the ligand.

A method of forming the platform assembly of the present invention is further disclosed, wherein the method includes the steps of providing a platform material having a plurality of pins on the surface of the platform material; and attaching a ligand to the pins, the ligand being reactive with a target reagent to form a complex when placed in contact therewith, the ligand portion of the complex remaining substantially attached to the pins during an analytic measurement. The pins may be formed of a material suitable for direct chemical or adsorptive attachment of the ligand to the pins. The method may further include the step of at least partially coating the pins with a ligand attachment-promoting material.

### 4. Brief Description of the Drawings

Figure 1 is a schematic representation of the platform assembly of the invention. In this figure, pins extending from the platform have been adapted to be insertable into a multi-well plate containing a target reagent. The pins are shown as including a ligand directly attached thereto by covalent or adsorptive means, the ligand being bound to a target reagent (Δ).

Figure 2 is a schematic representation which illustrates a pin of the platform assembly of the present invention during various steps of an analytical method.

Figure 2A shows a pin pre-coated with a ligand, such as an antigen, following insertion into a well containing a test substance, such as test antibody.

Figure 2B shows the pin from Fig. 2A following reaction of the antigen with a test antibody.

Figure 2C illustrates the pin from Fig. 2B following further reaction with a labeled anti-idiotype antibody which results in a detectable product on the surface of the pin.

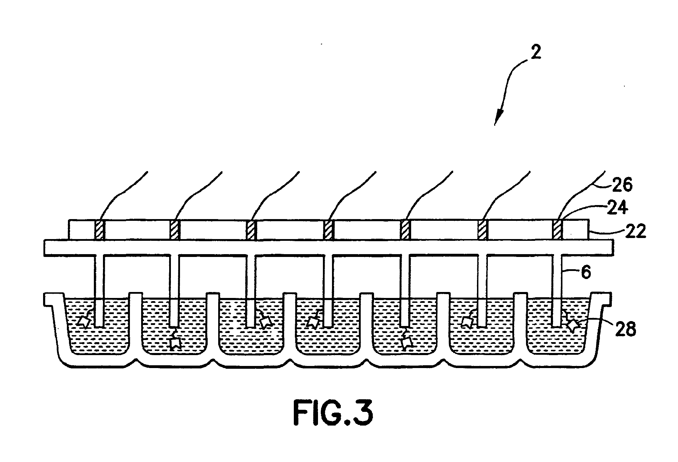

Figure 3 is an embodiment of the invention where the platform assembly further includes a connector plate positioned on the platform, the connector plate including a base plate and a plurality of connectors connected to the pins for facilitating the transmission and/or receipt of a signal to and from the pins. Electrical leads are shown in contact with the connectors. These may lead to an outside instrumentation source for real-time monitoring and control of a reaction occurring on the pins or, alternatively, in the wells of the microtiter plate. Each well may include a unique test substance for screening of a biological activity, such as the ability to bind to the ligand attached to the pin.

### 5. Detailed Description of the Invention

The principal object of the present invention is to modify conditions for carrying out analytical and diagnostic methods typically performed using microtiter plates. This is accomplished by use of the platform assembly of the present invention which is useful for conducting analytical and diagnostic assays. These assays include, but are not limited to, drug screening assays in which unknown compounds are tested for a particular biological activity.

The platform assembly for conducting analytical measurements includes (a) a platform; and (b) a plurality of pins extending from the platform, the pins being adapted to be insertable into a reagent repository and the pins including a ligand attached thereto, the ligand being reactive with a target reagent when placed in contact therewith to form a complex, the ligand portion of the complex remaining substantially attached to the pins during an analytic measurement.

A complex is herein defined as a combination of two or more substances. By "substantially attached" it is meant that all or nearly all of the ligand portion of the complex remains attached to the pin following complex formation and during an analytical measurement. By "during an analytical measurement" it is meant during a period of time sufficient to produce a measurable signal.

Reagent repositories may include test tubes, vials, trays, or wells. In one preferred embodiment, the reagent repository corresponds to the wells of a multi-well plate such as a microtiter plate. The multi-well plate may, alternatively, include virtual wells. Microtiter-like plates including virtual wells have surfaces which are patterned to have relatively hydrophilic domains within relatively hydrophobic fields so that a sample is physically constrained by surface tension to the more hydrophilic domains by the edges of the more hydrophobic fields. It is the arrangement of hydrophilic domains within hydrophobic fields which creates "virtual wells". Microtiter-like plates including virtual wells are known in the art.

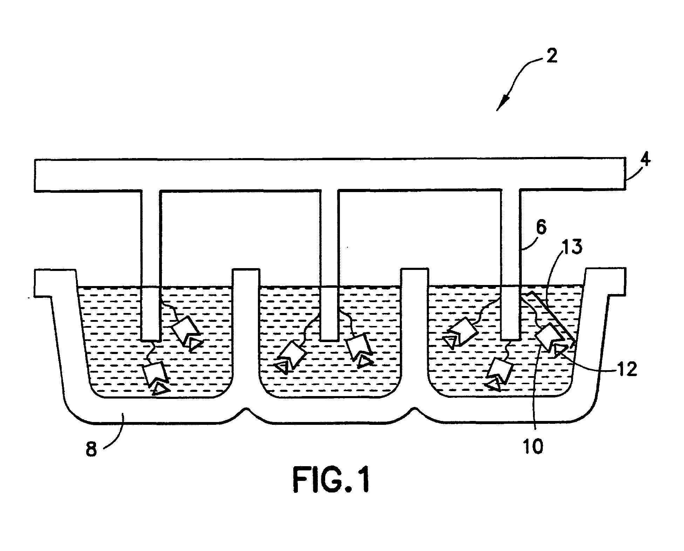

Referring now to Figure 1, which shows the platform assembly 2 according to the present invention, the assembly includes a platform 4 and a plurality of pins 6 extending from platform 4, the pins being adapted to be insertable into a reagent repository 8 and the pins including a ligand 10 attached thereto wherein ligand 10 is reactive with a target reagent 12 when placed in contact therewith to form a complex 13. The liquid portion of the complex remains attached to the pin during an analytical measurement. In the particular embodiment shown in Figure 1, the reagent repository corresponds to the wells of a microtiter plate. In this instance, the pins are adapted for insertion into the wells of the microtiter plate by being formed on the platform in a spatial arrangement approximating the spatial arrangement of the plurality of wells in the microtiter plate. It is noted that it is possible with the platform assembly according to the present invention to place different ligands on the pins. It is further possible to place different reagents in the wells of the microtiter plate.

As described above, the pins of the present invention include a ligand attached thereto. In one embodiment, the ligand is chemically attached directly to the pin surface. In a further embodiment, the ligand is directly adsorbed to the pin surface. For purposes of this invention, the ligand is attached to the pins by a means other than only surface tension.

Suitable ligands for chemical or adsorptive attachment to the pins include proteins or nucleic acids. Nucleic acid ligands may include naturally occurring or chemically synthesized forms of DNA and RNA, as well as any modified or mutated forms thereof. Moreover, chemically synthesized oligonucleotides or oligonucleotides derived from a natural, modified, or mutant form of DNA are suitable ligands for attachment to the pins. Chemical synthesis of various nucleic acid forms may be performed by methods known in the art, such as those described by Caruthers in *Science,* volume 230: 281-285 (1985) and *DNA Structure, Part A: Synthesis and Physical Analysis of DNA*, Lilley, D.M.J. and Dahlberg, J.E. (Eds.), Methods Enzymol., volume 211, Academic Press, Inc., New York (1992).

As was aforementioned, suitable ligands include proteins. Moreover, modified or mutated proteins or protein fragments may be bound to the pins. For example, in one embodiment, the ligand is a peptide or polypeptide. Suitable methods for synthesizing polypeptides are described by Stuart and Young in "Solid Phase Peptide Synthesis," 2^{nd} edition, Pierce Chemical Company (1984), and Solid Phase Peptide Synthesis, Methods Enzymol., volume 289, Academic Press, Inc., New York (1997).

Ligands may be attached directly to the surface of the pins. In an alternative embodiment, the pins further include a ligand attachment-promoting material associated therewith. For example, the pins may be coated or partially coated with an avidin solution to attach specific ligands that contain a biotin tag. Such coating may be by such means as spraying, dipping or painting. Moreover, the ligand may be present in a composition which includes a ligand attachment-promoting material. The ligand may be attached to the surface of the pin *via* a linker molecule. The presence of an anchored ligand on the pins facilitates washing and subsequent reactions with reagents located in the reagent repository.

Reagents in the reagent repository may be selected from the following: enzyme, antibody, antigen, receptor, dye, such as a fluorescent dye, ligand, such as a labeled ligand, cytokine, activator, inhibitor, buffer, cells and combinations thereof.

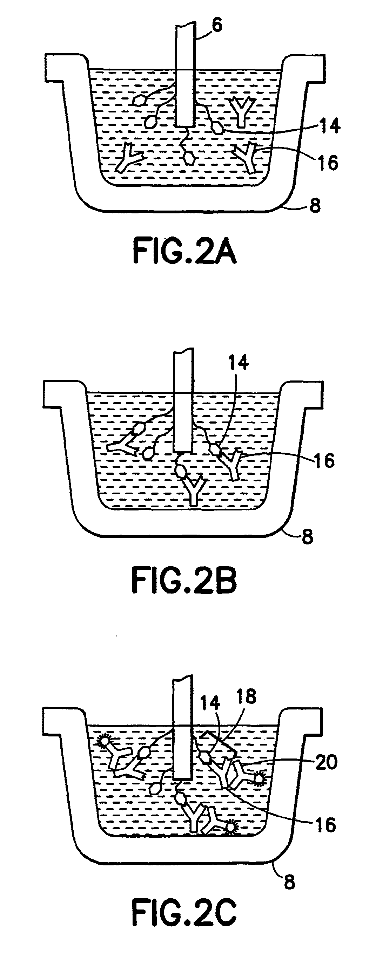

Referring now to Figure 2, the embodiments shown in Figures 2A-2C are schematic representations of a pin of the platform assembly of the present invention during various steps of an analytical or diagnostic assay. The assay format shown is a heterogeneous assay format, which is described in further detail below. In particular, Figure 2A shows the pin 6 pre-coated with a ligand 14, such as an antigen, following insertion into a reagent repository (well) 8 containing a target reagent 16, such as a test antiserum. Each reagent in a given well may correspond to a unique test substance for screening of a biological activity. Figure 2B shows the pin following reaction of ligand 14 with target reagent 16. The ligand reacts with the reagent during incubation, as desired. The pins may be subsequently washed and brought into contact with further reagents, as shown in Fig. 2C, as desired. Figure 2C shows the pin of Fig. 2B following further reaction of the complex 18 on the pin with a second reagent 20, such as a labeled anti-idiotypic antibody to facilitate the generation of a signal on the face of the pins. As shown, the ligand portion (14) of complex 18 remains substantially attached to the pins during the analytical method.

It is further contemplated that homogenous assays which do not require any sample separation steps may be performed using the platform assembly of the present invention. Such homogeneous assays are often used for DNA detection by energy transfer and fluorescence quenching. This fluorescent approach relies on strategically positioning a fluorescent label on one DNA probe and a second label on a second probe. The two probes may be attached to the same pin. The two probes hybridize to a target immediately downstream from one another at a fixed distance apart. A detectable change in one or both labels may be generated when the two probes concurrently hybridize to a target molecule in the reagent repository. A fluorescent label and a quenching label are generally used in these homogenous assays. It the two tags are separated by an appropriate distance when hybridizing to adjacent or closely-linked targets, energy can be transferred between the fluorescence label and the quenching label resulting in a detectable signal change. The presence of the target DNA in the reagent repository may be detected as a decrease in emission from fluorescence due to the quenching of emission by the quenching label. The primary advantage of the energy transfer and fluorescent quenching assays is assay simplicity. These approaches allow rapid assays since detection is performed in a single step and no reagent transfer steps are required. However, in general, homogeneous assays are less sensitive than heterogeneous assays and may lack detection levels required for many analytical and diagnostic assays.

For this reason, it is well within the contemplation of the present invention that the analytical measurement methods and screening methods encompassed by the present invention include heterogeneous assays. In this instance, the ligands attached to the pins may need to be brought into contact with a series of reagents with multiple washing and incubation steps as required in order to yield a detectable product.

It may be appreciated that any number of heterogeneous assay formats are possible. For example, in one indirect labeling scheme, a small molecule such as digoxigenin may be incorporated into an analyte located in a reagent repository. Following reaction of the analyte with the ligand bound to the pins, the presence of the label may be revealed using enzyme conjugates that specifically bind to the digoxigenin in the analyte (e.g. anti-digoxigenin conjugated to alkaline phosphatase). Thus, after initially dipping the pins into reagent repositories containing the analytes, the pins are washed, such as by dipping into a buffer solution in a reagent tray and are then dipped into a reagent repository containing the enzyme conjugate. The analyte may be visualized using a chemiluminescent substrate for the enzyme.

In another assay format, the ligand attached to the pins is a protein antigen. Following attachment of the ligand, non-specific sites on the pins may be blocked. Antibody test compounds located in separate wells of a microtiter plate are then brought into contact with the protein antigen by dipping the pins into the wells of the microtiter plate. Following additional washing of the pins by dipping in suitable wash solutions, the pins may be brought into contact with an anti-antibody compound which is labeled with a reporter molecule. Following additional washing, the immune complex including the reporter molecule, which is bound to the solid phase of the pin, may be detected. In this particular embodiment, which is shown in Fig. 2, a measurable product forms on the pin.

In an alternative embodiment, the measurable product may form in the reagent repository. For example, in an assay format most similar to an enzyme linked immunosorbent assay (ELISA), a protein antigen is first attached to the pin. Following washing of the pins antibody test compounds in wells of a microtiter plate react with the antigen. After additional washing, a ligand molecule is added which can detect the antibody, the ligand molecule being covalently coupled to an enzyme such as peroxidase. After free ligand is washed away, the enzyme-coupled ligand which has been bound to the pins is visualized by the addition of a chromagen. The chromagen does not bind to the pins, but is rather a colorless substrate located in the reagent repository which when acted upon by the enzyme portion of the enzyme-coupled ligand produces a colored end product. The presence of reactivity of the test antibody compounds with the protein antigen, as well as the amount of the test antibody, may be measured by assessing the amount of colored end product by optical density scanning of a microtiter plate, for example.

In one desired embodiment, the pins may include sensors of a signal correlating to an analytical measurement. For example, the pins may include a fiber optic sensor. Where the product forming on the pins emits some form of light, the fiber optic sensor may aid in a transmission of the light signal to an outside instrumentation source. This would provide for real-time monitoring of detectable product formation.

In a further embodiment of the present invention, the pins include transmitters of a signal which facilitates a reaction between the ligand and a given reagent. The transmitted signal may include, for example, heat, cooling, electricity, light and magnetism or any combination thereof. The ability to transmit a signal to the pins provides a means of controlling the reaction between the ligand attached to the pins and subsequent reagents with which it is in contact. Moreover, it is well within the contemplation of the present invention that a given transmitted signal may be the same for all the pins extending from the platform of the assembly or, alternatively, the transmitted signal may be different for different pins. Where the transmitted signal is heat or electricity, it is desirable that the pins include conductive materials such as, but not limited to, metal.

The pins may further include materials selected from the following: plastic, ceramic, glass, quartz, porcelain, and germanium. The materials can be used in pure form, as mixtures, alloys or blends. Both the pins and the platform from which the pins extend may be transparent. In this instance, it is desired that the pins and/or platforms be made of a transparent material such as quartz, glass, plastic, germanium or silicon, which are suitable for all visual tests such as microscopic, camera-assisted and laser-assisted tests.

Transparent plastics include the following: polystyrene, styrene/acrylonitrile, polypropylene, polycarbonate, polyvinyl chloride, poly(methylmethacrylate), polyesters, silicones, polyethyelene/acrylate, polylactide or cellulose acetate, cellulose propionate, cellulose butyrate and any mixtures thereof. It is noted that silicon or germanium supports are well suited for applications in which detection or induction of a reaction using near infrared light is necessary.

The platform assembly of the present invention is generally suitable for all analytical and diagnostic methods presently carried out in microtiter plates, such as those involving colorimetric, fluorimetric or densitometric measurements. For example, it is possible to use and measure light scattering, turbidity, fluorescence, luminescence, raman scattering, radioactivity, isotope labeling, pH shifts or ion shifts as well as wavelength-dependent light absorption alone or in combination. However, other measured properties are further contemplated by the present invention.

As described above, direct real-time monitoring of a reaction taking place on the face of the pin is possible where the pins are to include sensors of a signal correlating to an analytical measurement. To this end, in one embodiment of the invention, the platform assembly further includes a connector plate positioned on the platform, the connector plate including (i) a base plate and (ii) a plurality of connectors supported by the base plate and connected to the pins for facilitating the transmission and/or receipt of a signal to and from the pins. For example, electrical leads may lead from the connectors to an outside instrumentation source for direct recording of a signal being generated on the pins or in the reagent repository.

Referring now to Figure 3, a particular embodiment is shown wherein the platform assembly 2 according to the present invention, includes a base plate 22 and a plurality of connectors 24 supported by base plate 22 and connected to the pins 6. Electrical leads 26 provide contact between the connectors 24 and an outside instrumentation source for direct real-time monitoring and control of a reaction. Pins 6 may contain sensors of a signal being generated and/or transmitters of a signal to the pins. The signal transmitted may be heat, for example, which may allow a reaction to proceed quickly to generate a detectable signal. Moreover, since many widely used detection methods rely on measuring total fluorescence using optical filters to separate excitation and emission, it is within the contemplation of the present invention that a transmitted signal may correspond to light of a wavelength appropriate for excitation of a particular fluorescent label, whereas the signal received through the pins may correspond to light emitted from the label.

As shown in the specific embodiment in Figure 3, the same ligand 28 is attached to pins 6. Alternatively, one or more of the ligands bound to the pins of the assembly may be different. Each well of the multiwell plate in Figure 3 may contain a unique test compound for testing of a biological activity, such as the ability of the test compound to bind to ligand 28.

Analytical methods which may be carried out on the pins of the assembly of the invention include, but are not limited to the following: binding of antibodies to antigens, the specific cleavage of substrate molecules by enzymes, polymerase chain reactions (PCR), the interaction between receptors and ligands, the interaction between different or identical cell types such as enzyme assays, titration assays such as virus titration assays, erythrocyte or platelet aggregation, agglutination assays with latex beads, ELISA (enzyme-linked immunosorbent assay) or radioimmunoassays (RIA).

As described above, the invention provides an analytical measurement method including the steps of: providing a platform assembly including a platform and a plurality of pins which are adapted to be insertable into a reagent repository; attaching a ligand to the pins for conducting analytical measurements; contacting the attached ligand with a target reagent in a reagent repository to form a complex, the ligand portion of the complex remaining substantially attached to the pins during an analytic measurement; and measuring a signal which correlates with the analytical measurement. Where the reagent repository is a multi-well plate, it may be appreciated that each well may contain a unique reagent which reacts with or participates in a reaction with a ligand attached to the pin. Moreover, as described above, the same or different ligands may be bound to each of the pins.

In one embodiment, the analytical measurement method provided by the present invention may further include the step of determining a biological activity selected from the group consisting of increases or decreases in metabolites or ions, changes in the transcription of certain genes, changes in enzymatic activity, changes in pH, changes in cell growth, antibody binding, and interactions between receptors and ligands. The analytical measurement may be a colorimetric, fluorimetric or densitometric measurement.

Optical detection methods such as fluorescence detection generally employ an excitation and an imaging step. The excitation and imaging steps can each be done through the pin or through the top or through the bottom of a multi-well plate.

The present invention provides methods of high throughput screening that employ the inventive platform assembly. Moreover, non-high throughput screening methods are provided. These methods of screening include a means of identifying a substance capable of binding to or modulating the activity of a particular ligand including the steps of providing a platform assembly including a platform and a plurality of pins which are adapted to be insertable into a reagent repository; attaching a ligand to the pins; exposing the attached ligand to a substance suspected of being capable of binding to or modulating the activity of the ligand to form a complex, the ligand portion of the complex remaining substantially attached to the pins during the screening process; and determining whether the substance binds to or modulates the activity of the ligand. In one embodiment, the screening method further includes the step of at least partially coating the pins with a ligand attachment-promoting material.

The platform assembly of the present invention can be made my numerous methods. In general, these methods include the steps of (a) providing a platform material having a plurality of pins on the surface of the platform material; and (b) attaching a ligand to the pins, the ligand being reactive with a target reagent to form a complex when placed in contact therewith, the ligand portion of the complex remaining substantially attached to the pins during an analytic measurement. Moreover, the method may include the step of at least partially coating the pins with a ligand attachment-promoting material. In preferred embodiments, the platform material is selected from the following: plastics, metal, ceramic, glass, quartz, porcelain, germanium and combinations thereof. The pins may be formed of similar materials. It is noted that one or more of the pins may be formed of a different material as compared to other pins in the array.

In particular embodiments, a plurality of pre-formed pins is attached to the surface of the platform material. For example, pre-formed pins may be inserted into a carrier such as, but not limited to, an epoxy resin plate, metal plate, plastic sheet, or other formed planar substrate. The pins, in particular, can be inserted with a non-slip fit or a slip fit, the slip fit being tight enough to align the pins but permitting slippage of the pins when a force is applied. When a slip fit is used, the pin is preferably held in the carrier by an enlargement at one end of the pin such that the pins will not fall out of the carrier. Use of the slip fit may allow the pins to be used in a disposable fashion, if so desired. The slip fit further permits the pins to adjust their lengths to the surface defined by the reagent repository, such as the wells of a microtiter plate.

In other methods, the plurality of pins may be formed by one of the following methods: machining the pins into the surface of the platform material, laser cutting the pins into the surface of the platform material, and molding the pins from the platform material. Molding from a suitable platform material such as plastic, glass, and metal may be accomplished by conventional macroscopic methods or standard micro-fabrication methods used in micro-machining, such as x-ray, lithography, electrodisposition, and molding.

Moreover, other methods for forming the platform assembly according to the present invention may include milling of plastics using a computer numerical control machine, casting molten material into an open mold, injection molding, and cold forging of a relatively soft metal having a low melting point. In cold forging, the metal that is being cold forged is subjected to intense pressure upon sudden impact with a very heavy mold. Furthermore, provided the platform and pins are formed of metal or other conductive materials, a preferred method for forming the platform assembly according to the present invention is that of electrical discharge machining wherein metal is removed from a plate, such as a metal plate, in order to form the plurality of pins extending therefrom.

With reference now to the particular advantages of performing analytical and diagnostic assays using the platform assembly according to the present invention, it is noted that the pins allow for direct monitoring and control of the reaction and in real-time. Assays utilizing the platform assembly provided by the invention allow for more versatility than typical assay formats where ligands are attached to a well surface. For example, the plurality of pins may represent an array of different ligands. Furthermore, in specific embodiments where the reagent repository is corresponding to wells of a microtiter-like plate, each reagent in the spatial array of wells can be a different fluid, either due to the nature of the liquid part of the fluid or due to the nature of compounds dissolved in the liquid, or both. Finally, each pin in the spatial array of pins extending from the platform can be used to transmit and/or receive a unique signal. All of these factors combined provides versatility in terms of the number of compounds which may be tested, the type and number of biological activities which may be assessed, and the control and monitoring of reactions.

Moreover, in typical microtiter assay formats currently used, the reaction time is dependent on proper mixing of the reagents, which is a diffusion-limited process. In contrast, when the pins of the platform assembly of the present invention are dipped in a reagent repository, the ligand and other compounds which may be bound thereto are immediately available for reaction with a given reagent in the reagent repository.

Furthermore, the use of the pins allows for the use of smaller volumes of reagents. This is desirable for a number of reasons. For example, it allows for the conservation of scarce biological and chemical materials. To this end, assays may be developed at a faster pace due to the requirement for less reagent purification. Furthermore, the conservation of test compounds allows for more than one assay to be performed on a given test compound. This would allow one to assess more than one aspect of a given test substance's biological activity. Finally, it is noted that by performing reactions on the surface of the pins, increased sensitivity of the reaction may be achieved due to the increased concentration of analytes on the pin's surface, which would result in an increase in the signal intensity.

## Claims

1. A platform assembly for conducting analytical measurements comprising
(a) a platform; and
(b) a plurality of pins extending from said platform, said pins being adapted to be insertable into a reagent repository and said pins including a ligand attached thereto, said ligand being reactive with a target reagent to form a complex when placed in contact therewith, the ligand portion of the complex remaining substantially attached to the pins during an analytic measurement.

2. The assembly of claim 1 wherein said pins further include a ligand attachment-promoting material associated therewith.

3. The assembly of claim 1 wherein said ligand comprises a composition which includes a ligand attachment-promoting material.

4. The assembly of claim 1 wherein said ligand is chemically attached to said pin.

5. The assembly of claim 1 wherein said ligand is adsorbed to said pin.

6. The assembly of claim 1 wherein said ligand is attached to the same or different pins.

7. The assembly of claim 1 wherein said ligand is a protein or nucleic acid.

8. The assembly of claim 1 wherein said ligand is a peptide or polypeptide.

9. The assembly of claim 1 wherein said ligand includes a reporter molecule.

10. The assembly of claim 1 wherein said reagent repository is a multiwell plate.

11. The assembly of claim 1 further comprising a connector plate positioned on said platform, said connector plate including (i) a base plate and (ii) a plurality of connectors supported by said base plate and connected to said pins for facilitating the transmission and/or receipt of a signal to and from said pins.

12. An analytical measurement method which comprises the steps of:
(a) providing a platform assembly comprising a platform and a plurality of pins which are adapted to be insertable into a reagent repository;
(b) attaching a ligand to the pins for conducting analytical measurements;
(c) contacting the attached ligand with a target reagent in a reagent repository to form a complex, the ligand portion of the complex remaining substantially attached to the pins during an analytic measurement; and
(d) measuring a signal which correlates with said analytical measurement.

13. The method of claim 12 wherein said method further includes the step of determining a biological activity selected from the group consisting of changes in levels of metabolites or ions, changes in the transcription of certain genes, changes in enzymatic activity, changes in pH, changes in cell growth, antibody binding, and interactions between receptors and ligands.

14. The method of claim 12 wherein said analytical measurements are selected from the group consisting of colorimetric, fluorometric or densitometric measurements.

15. The method of claim 12 further including the step of at least partially coating said pins with a ligand attachment-promoting material.

16. A method of screening to identify a substance capable of binding to or modulating the activity of a ligand comprising the steps of:
(a) providing a platform assembly comprising a platform and a plurality of pins which are adapted to be insertable into a reagent repository;
(b) attaching a ligand to said pins;
(c) exposing said attached ligand to a substance suspected of being capable of binding to or modulating the activity of said ligand to form a complex, the ligand portion of the complex remaining substantially attached to the pins during the screening process, wherein said substance is in said reagent repository; and
(d) determining whether said substance binds to or modulates the activity of said ligand.

17. The method of claim 16 further including the step of at least partially coating said pins with a ligand attachment-promoting material.
